# EUROPEAN PATENT APPLICATION

(11) **EP 4 190 284 A1**
(43) Date of publication of application: **07.06.2023**
(21) Application number: 21851425.5
(22) Date of filing: 05.07.2021
(51) Int. Cl.: A61F 2/82, A61M 29/04, A61B 17/11

(54) **LUMEN ANASTOMOSIS SUPPORT DILATOR**

(30) Priority: 28.07.2020 CN 202010735867; 28.07.2020 CN 202021522078 U
(71) Applicant: Sparkle Medical Equipment (Shanghai) Co., Ltd., Shanghai 200335 (CN)
(72) Inventor: WU, Jiong, Shanghai 200335 (CN); LAW, Man Chuen, Shanghai 200335 (CN); WANG, Qiang, Shanghai 200335 (CN); DING, Yi, Shanghai 200335 (CN)
(74) Representative: Haseltine Lake Kempner LLP
(86) International application number: PCT/CN2021/104574
(87) International publication number: WO 2022/022235

(57) **Abstract**

A lumen anastomosis support dilator, comprising: a support main body (1), the support main body (1) being made of an elastic material or a shape memory material and configured to be at least partially placed in a lumen (101) to stretch a lumen wall (100); and a connecting rod (2), the connecting rod (2) being connected to the support main body (1). During anastomosis, a surgeon can grip the support main body (1) with an instrument and place the support main body (1) in the lumen (101), and after the instrument is released, the support main body (1) made of an elastic material or a shape memory material can restore and appropriately hold open the lumen (101). Therefore, the lumen anastomosis support dilator can quickly dilate the lumen (101) to assist in a surgical operation, thereby reducing damage to the lumen wall (100) and improving the efficiency and success rate of anastomosis for the lumen (101).

## Description

This application claims the priority of Chinese patent application 2020215220787 with an application date of July 28, 2020, and the priority of Chinese patent application 2020107358677 with an application date of July 28, 2020. This application cites the full text of the above Chinese patent application.

### TECHNICAL FIELD

The invention relates to the field of auxiliary instruments for lumen anastomosis in medical surgical operations, in particular a lumen anastomosis support dilator.

### BACKGROUND

Lumen anastomosis is a basic surgical form in clinical, including lumen damage repair, lumen transplantation, lumen overlap, lumen suture and repair, etc. It can be used for blood vessels, lymphatic channels, vas deferens, biliary tract, trachea, bronchi, etc. It is an effective treatment method commonly used to reconstruct lumen function. It is widely used in all kinds of surgical operations, such as flap transplantation, replantation of severed limbs, accidental vascular injury, organ transplantation, arteriovenous internal fistula anastomosis, lymphatic channels anastomosis, vas deferens anastomosis, biliary tract anastomosis, trachea anastomosis and so on. With the continuous development of micro surgical instruments, micro sutures and operating microscopes, manual small (micro) lumen anastomosis technology has gradually matured, and the diameter of the anastomotic lumen is getting thinner and thinner. However, even for experienced surgeons, it is not easy to complete a perfect lumen anastomosis, especially the small micro lumen anastomosis mentioned above.

First of all, the anastomotic stomas of two broken ends opposite each other of the lumen should be accurately aligned without torsion, stenosis, eversion or varus, which means that the two broken ends are required to be aligned intima to intima and muscularis to muscularis. This way, the healing scar is the least, and the patency rate is also increased. Therefore, it is required that the diameters of the two broken ends should be equal as far as possible, and the dilators with equal diameters or tweezers dedicated to dilating the lumen can be used to dilate the lumen. However, the paradox is that for some small lumens, non-invasive techniques are attached great importance, and tweezers are not allowed to clamp the intima and muscularis of the lumen to be anastomosed, otherwise the patency rate will be reduced. Flexible or non-invasive internal support is required to achieve anastomosis of the anastomotic stoma.

Secondly, taking the blood vessel as an example, the blood vessel is a kind of lumen, for anastomosis of blood vessel, the blood vessel needs to be exposed and the blood flow can be controlled to provide a bloodless field of view to complete the surgical operation; according to the size and anatomical position of the blood vessel, different types of non-invasive of blood vessel forceps, soft and elastic colloidal strips, or balloon catheters are used to block blood flow through the lumen of the blood vessel. After blocking the blood flow, along with the elastic retraction and collapse of the vascular wall, the lumen becomes smaller or even occluded, and the anterior and posterior walls often fit together and are not easily separated, causing great obstacles to the suture operation, and the suture can easily pierce the contralateral blood vessel wall. The operation is difficult, inefficient, and time-consuming, resulting in a long blood flow blocking time and a high risk. Some surgeons will make some self-made packing materials to assist suture. Although some problems can be solved, the efficiency is still low, the function is single, and it is not standardized.

### CONTENT OF THE PRESENT INVENTION

The technical problem to be solved by the present invention is to provide a lumen anastomosis support dilator in order to overcome the problems in the lumen suturing technology in the prior art.

The present invention solves the above technical problems by means of the following technical solutions:
A lumen anastomosis support dilator is characterized by comprising:
a support main body, the support main body is made of an elastic material or a shape memory material and is used for partially placed in a lumen at least to dilate a lumen wall;
a connecting rod, the connecting rod is connected to the support main body.

In this solution, during anastomosis operation, a surgeon can grip the support main body with an instrument (such as micro forceps, needle holders, etc.) and place the support main body in the lumen, and after the instrument is released, the support main body made of an elastic material or a shape memory material can restore and appropriately hold open the lumen, which is convenient for doctors to perform subsequent anastomosis operation. When a part of the anastomosis is completed, the support main body can be taken out through the unstitched gap by using the connecting rod, and the subsequent stitch can be continued. Therefore, the lumen anastomosis support dilator can quickly dilate the lumen to assist in a surgical operation, thereby reducing damage to the lumen wall and improving the efficiency and success rate of anastomosis for the lumen.

The above elastic material can be rubber (including but not limited to polyisoprene, styrene-butadiene rubber, butadiene rubber, isoprene rubber, neoprene rubber, butyl rubber, etc.), silicone rubber (including but not limited to methyl silicone rubber, methyl vinyl silicone rubber, methyl phenyl silicone rubber, methyl vinyl phenyl silicone rubber, nitrile silicone rubber, fluorosilicone rubber, etc.), polyurea, latex and so on. Shape memory materials may include shape memory alloy materials and shape memory polymer materials. Shape memory alloy materials include but are not limited to nickel-titanium alloy, titanium-nickel-copper, titanium-nickel-iron, titanium-nickel-chromium, copper-nickel alloy, copper-aluminum alloy, copper-zinc alloy, copper-zinc-aluminum alloy, iron alloy, iron-silicon carbon alloy, iron-nickel alloy, iron-manganese alloy, iron-manganese carbon alloy, iron-manganese-silicon alloy. Shape memory polymers include, but are not limited to, polyvinyl alcohol, polycaprolactone, polylactic acid, polydioxanone, polyisoprene, cross-linked polyethylene, polynorbornene, styrene-butadiene copolymers, polyamides, polyethylene terephthalate, polyacrylic acid, polymethacrylic acid, polyacrylamide, poly(N-isopropylacrylamide), epoxy polymers, polydimethylsiloxane, polyaniline, polyurethane, poly-L-lysine, poly-L-glutamic acid, collagen, alginate, hyaluronic acid, chitosan, starch, cellulose, and copolymers of the above.

Preferably, the connecting rod is provided with a plurality of the support main bodies.

In this solution, a plurality of support main bodies may be applicable to a plurality of lumens, and the plurality of support main bodies may have a plurality of different outer diameters, so one lumen anastomosis support dilator may be applicable to lumens with different diameters.

Preferably, the two ends of the connecting rod are each provided with the support main body.

In this solution, the two support main bodies can be placed in the two lumens to be anastomosed, so that the anastomosis of two lumens can be achieved with one lumen anastomosis support dilator, making the two lumens relatively fixed during stitching, facilitating the stitching operation and reducing the number of equipment needed during operation.

Preferably, the support main body is a rotary body.

In this solution, since the cross section of the lumen is mostly circular, the support main body is a rotary body that can be better adapted to the lumen to facilitate the anastomosis operation.

Preferably, the support main body comprises a main body part, at least one end of the main body part is provided with a tapered part, and the outer diameter of the tapered part decreases outward from the main body part, and the maximum outer diameter of the main body part is larger than the maximum outer diameter of the tapered part;
and/or, the included angle between the axis lines of the two support main bodies at both ends of the connecting rod is 0-180°.

In this solution, the main body part has a larger outer diameter and can be used to support the lumen, and the tapered part can be in the shape of a cone, a circular truncated cone, etc., and the tapered part can be easily inserted into the lumen, so as to make it easier for the entire support main body to enter the lumen. The support main body can be provided with only one tapered part or one tapered part at each end of the support main body to enable the support main body to be inserted into the lumen from both directions. That is, the shape of the support main body can be a large outer diameter in the middle and small outer diameter at both ends, or a large outer diameter at one end and a small outer diameter at the other end. Specifically, the support main body may be spherical, hemispherical, ellipsoidal, semi-ellipsoidal, conical, circular truncated cone, waterdrop shape, rugby, gourd, and the similar shape.

Since the openings of the two lumens to be stitched may have different relative positions, the axis lines of the two support main bodies at both ends of the connecting rod can be set to be collinear, parallel, or at other angles to suit lumen anastomosis requirement for different cases.

Preferably, the maximum outer diameters of the main body parts of each support main body are same or different.

In this solution, the main body parts of two support main bodies on the same connecting rod may have the same maximum outer diameter to apply to the anastomosis between lumens with the same outer diameters. In addition, the two support main bodies can also have different maximum outer diameters, so as to be suitable for anastomosis between lumens with different diameters.

Preferably, the lumen anastomosis support dilator further comprises an operating rod, one end of the operating rod is connected to the middle of the connecting rod.

In this solution, the support main body can be pushed and pulled by the operating rod to adjust the position and angle of the support main body, and the user can use the operating rod to take out the support main body from the lumen.

Preferably, the connecting rod is able to be bent, so that the relative positions between the different support main bodies can be adjusted.

In this solution, since the connecting rod can be bent, when the support main body is taken out, the position and angle of the support main body can be automatically adjusted to avoid damage to the lumen. Moreover, the connecting rod can be bent into any angle, which can be applied to different anastomosis operations. The connecting rod can be a straight rod, or, due to the needs of specific anastomotic operations, the connecting rod can also be pre-molded into a curved tube. The connecting rod can be made of the same material as the support main body, and further, the connecting rod can be integrally-formed with the support main body. The connecting rod and the support main body can also be made of different materials, and those skilled in the art can select materials according to actual conditions. Further, the connecting rod and the support main body can be integrally-formed, but not limited to integral forming.

Preferably, the support main body is provided with a hollow cavity, and one end of the support main body is provided with a through hole communicating with the hollow cavity; or, two ends of the support main body are respectively provided with through holes communicating with the hollow cavity.

In this solution, the support main body may have a hollow cavity so that the support main body can be easily compressed. The support main body is provided with a through hole to facilitate the molding of the hollow cavity. If both ends of the support main body are opened with through holes, the hollow cavity is penetrating, so the fluid in the lumen can continue to keep flowing through the hollow cavity during the anastomosis operation, which can solve the problem of collapse of the lumen wall affecting the stitching due to interruption of the fluid flowing inside the lumen during the lumen stitching. There may be one or more through holes at one or both ends of the support main body. It can be understood that, the support main body may not be provided with a hollow cavity, so that the support main body has a greater supporting force.

Preferably, the connecting rod is provided with a fluid channel, and the fluid channel is communicated with the hollow cavity of the support main body.

In this solution, the fluid in the lumen can flow in the hollow cavity, the fluid in the hollow cavity can be drained to a preset position through the fluid channel, and the fluid channel can be set according to the actual situation to facilitate the anastomosis operation. Specifically, one end of the hollow cavity can be communicated with the fluid channel, so the fluid flowing into the hollow cavity is completely discharged from the fluid channel, or the fluid in the hollow cavity is completely injected from the fluid channel. In addition, when a plurality of support main bodies is provided on the connecting rod, the hollow cavities of each support main body can be communicated through a fluid channel, during the stitching operation, different support main bodies are arranged in different lumens, and the fluid in one lumen can flow into another lumen through the hollow cavity and fluid channel, so as to realize the communication between different lumens during the stitching operation.

The positive improvement effect of the present invention is that the lumen anastomosis support dilator can be applied to lumen anastomosis operations, such as anastomosis of blood vessels, lymphatic channels, vas deferens, biliary tract, trachea, bronchi and other lumens. A surgeon can grip the support main body with an instrument (such as micro forceps, needle holders, etc.) and place the support main body in the lumen, and after the instrument is released, the support main body made of an elastic material or a shape memory material can restore and appropriately hold open the lumen, which is convenient for doctors to perform subsequent anastomosis operation. Since the support main body has a through hollow cavity, during the anastomosis operation, the fluid in the lumen can continue to flow through the hollow cavity, which can solve the problem that the stitching is affected by the collapse of the lumen wall caused by the interruption of the fluid flowing in the lumen during the stitching process. When a part of the suturing is completed, the support main body can be taken out through the unstitched gap by using the connecting rod, and the subsequent suturing can be completed. Therefore, the lumen anastomosis support dilator can quickly dilate the lumen and maintain the dilated state during the lumen anastomosis, so as to assist in a surgical operation, reduce damage to the lumen wall, and improve the efficiency and success rate of anastomosis for the lumens.

### BRIEF DESCRIPTION OF THE DRAWINGS

FIG. 1 is a schematic structural diagram of a lumen anastomosis support dilator according to Embodiment 1 of the present invention.
FIG. 2 is a schematic diagram of the internal structure of the lumen anastomosis support dilator according to Embodiment 1 of the present invention.
FIG. 3 is a schematic diagram of the lumen anastomosis support dilator according to Embodiment 1 of the present invention applied to end-to-end anastomosis.
FIG. 4 is a schematic diagram of the lumen anastomosis support dilator according to Embodiment 1 of the present invention applied to end-to-side anastomosis.
FIG. 5 is a schematic diagram of the lumen anastomosis support dilator according to Embodiment 1 of the present invention applied to side-to-side anastomosis.
FIG. 6 is a schematic structural diagram of a lumen anastomosis support dilator according to Embodiment 2 of the present invention.
FIG. 7 is a schematic diagram of the lumen anastomosis support dilator according to Embodiment 2 of the present invention applied to end-to-end anastomosis.
FIG. 8 is a schematic diagram of a deformed structure of the lumen anastomosis support dilator of FIG. 6.
FIG. 9 is a schematic diagram of a deformed structure of the lumen anastomosis support dilator of FIG. 8.
FIG. 10 is a schematic diagram of another deformed structure of the lumen anastomosis support dilator of FIG. 6.
FIG. 11 is a schematic diagram of the lumen anastomosis support dilator of FIG. 10 applied to end-to-end anastomosis.
FIG. 12 is a schematic diagram of the lumen anastomosis support dilator according to Embodiment 3 of the present invention applied to end-to-side anastomosis.
FIG. 13 is a schematic diagram of a deformed structure of the lumen anastomosis support dilator of FIG. 12 applied to end-to-side anastomosis.
Fig. 14 is a schematic diagram of another deformed structure of the lumen anastomosis support dilator of Fig. 13 applied to end-to-side anastomosis.
FIG. 15 is a schematic diagram of a deformed structure of the lumen anastomosis support dilator of FIG. 14.
FIG. 16 is a schematic diagram of the lumen anastomosis support dilator of FIG. 15 applied to end-to-side anastomosis.
FIG. 17 is a schematic diagram of the lumen anastomosis support dilator according to Embodiment 4 of the present invention applied to side-to-side anastomosis.
FIG. 18 is a schematic diagram of a deformed structure of the lumen anastomosis support dilator of FIG. 17 applied to side-to-side anastomosis.
FIG. 19 is a schematic diagram of another deformed structure of the lumen anastomosis support dilator of FIG. 17 applied to side-to-side anastomosis.
FIG. 20 is a schematic structural diagram of a deformed structure of the lumen anastomosis support dilator of FIG. 19.
FIG. 21 is a schematic diagram of the lumen anastomosis support dilator of FIG. 20 applied to side-to-side anastomosis.

### Brief description of reference signs

support main body 1
hollow cavity 11
tapered part 12
main body part 13
through hole 14
connecting rod 2
fluid channel 21
operating rod 3
lumen wall 100
lumen 101

### DETAILED DESCRIPTION OF THE SPECIFIC EMBODIMENTS

The present invention is further described below by means of embodiments, but the present invention is not limited to the scope of the embodiments.

For a better understanding of the present invention, the three most commonly used types of lumen suturing technique are listed below:
1. End-to-side anastomosis
   End-to-side anastomosis is widely used for clinical bypass grafting. Generally, a vertical straight line incision or an oval incision is made in the recipient lumen, and the end face of the donor lumen is trimmed into a bevel, and the length of the donor lumen is greater than the diameter of the recipient lumen.
2. End-to-end anastomosis
   The end faces of the two stitched lumens 101 should be the same as possible, or they can be trimmed into bevels to expand the area of the anastomotic stomas. Or use internal support to obtain similar stitched end faces.
3. Side-to-side anastomosis
   For side-to-side anastomosis, the two lumens to be anastomosed are usually approached, and a special lumen clamp is used to clamp the side wall of the anastomosis site to make a corresponding vertical incision.

### Embodiment 1

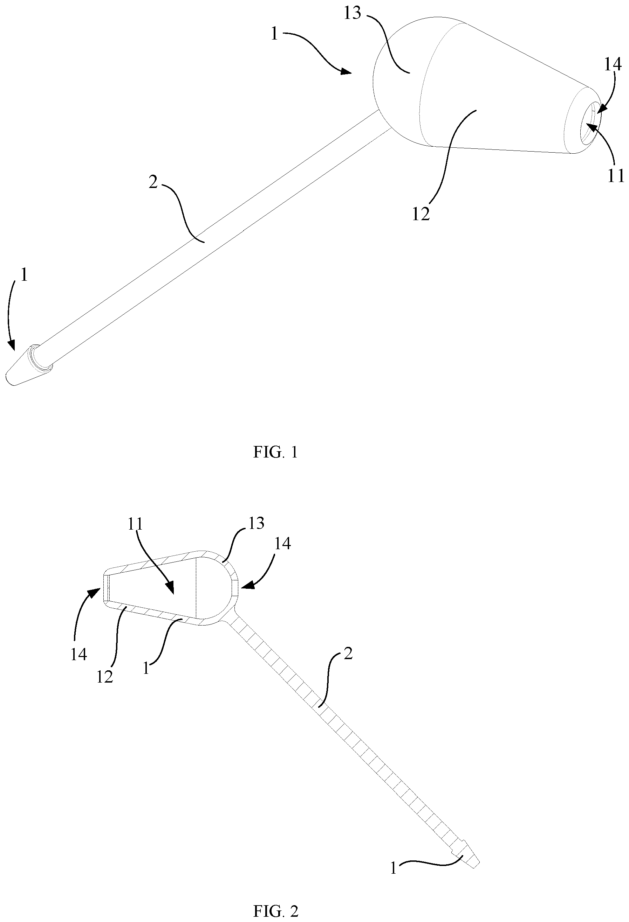

As shown in FIG. 1 to FIG.5, the lumen anastomosis support dilator according to embodiment 1 of the present invention includes a support main body 1 and a connecting rod 2. The support main body 1 is made of an elastic material or a shape memory material, and it can self-expand to its initial shape even if it is deformed, that is, it can provide good radial support, the flexible outer surface does not damage the inner surface of the lumen, and it can be easily retracted under the griping of special instruments. The support main body 1 is configured to be at least partially placed in a lumen 101 to dilate a lumen wall 100, and the support main body 1 is provided with a through hollow cavity 11; the connecting rod 2 is attached to the support main body 1.

The above elastic materials can be rubber (including but not limited to polyisoprene, styrene-butadiene rubber, butadiene rubber, isoprene rubber, neoprene rubber, butyl rubber, etc.), silicone rubber (including but not limited to methyl silicone rubber, methyl vinyl silicone rubber, methyl phenyl silicone rubber, methyl vinyl phenyl silicone rubber, nitrile silicone rubber, fluorosilicone rubber, etc.), polyurea, latex and so on. Shape memory materials may include shape memory alloy materials and shape memory polymer materials. Shape memory alloy materials include but are not limited to nickel-titanium alloys, titanium-nickel-copper, titanium-nickel-iron, titanium-nickel-chromium, copper-nickel alloys, copper-aluminum alloys, copper-zinc alloys, copper-zinc-aluminum alloys, iron-based alloys, iron-silicon alloys Carbon alloys, iron-nickel alloys, iron-manganese alloys, iron-manganese-carbon alloys, iron-manganese-silicon alloys. Shape memory polymer materials include but are not limited to polyvinyl alcohol, polycaprolactone, polylactic acid, polydioxanone, polyisoprene, cross-linked polyethylene, polynorbornene, styrene-butadiene olefin copolymer, polyamide, polyethylene terephthalate, polyacrylic acid, polymethacrylic acid, polyacrylamide, poly(N-isopropylacrylamide), epoxy polymer, polydimethylsiloxane, Polyaniline, polyurethane, poly-L-lysine, poly-L-glutamic acid, collagen, alginic acid, hyaluronic acid, chitosan, starch, cellulose and copolymers of the above substances.

During anastomosis operation, the surgeon can use instruments (such as micro forceps, needle holders, etc.) to grip the support main body 1 and place the support main body 1 in the lumen 101. When the instruments are released, the support main body 1 made of an elastic material or a shape memory material can restore and provide sufficient radial support to hold open the lumen wall 100 for the surgeon to perform subsequent anastomosis operations. When a part of the stitching is completed, the support main body 1 can be taken out through the unstitched gap by using the connecting rod 2, and the subsequent stitching can be continued. Therefore, this lumen anastomosis support dilator can rapidly dilate the lumen 101, so as to assist in surgical treatment, reduce damage to the lumen wall 100, and improve the efficiency and success rate of anastomosis for the lumens.

As shown in FIG. 1 and FIG. 2, the support main body 1 may have a hollow cavity 11, and one or more through holes 14 communicating with the hollow cavity 11 may be provided at one or both ends of the support main body 1. In this embodiment, both ends of the support main body 1 are provided with through holes 14 communicating with the hollow cavity 11, so during the anastomosis operation, the fluid in the lumen 101 can continue to flow through the hollow cavity 11, which can solve the problem of the wall collapse of the lumen 101 affecting the stitching due to interruption of fluid flow inside the lumen 101 during stitching of the lumen 101.

In some other embodiments, the support main body 1 may not be provided with the hollow cavity, that is, the support main body 1 is solid, so that the support main body 1 has a large supporting force.

Since the cross section of the lumen is mostly circular, the support main body 1 is preferably a revolving body, so that the support main body 1 can be better adapted to the lumen 101 to facilitate anastomosis.

The support main body 1 includes a main body part 13, at least one end of the main body part 13 is provided with a tapered part 12, the outer diameter of the tapered part 12 decreases outward from the main body part 13, and the maximum outer diameter of the main body part 13 is greater than the maximum outer diameter of the tapered part 12. The main body part 13 has a larger outer diameter and can be used to support the lumen 101, the tapered part 12 can be in the shape of a cone, a circular truncated cone, etc., the tapered part 12 can be easily inserted into the lumen, so as to make it easier for the entire support main body 1 to enter the lumen 101. The support main body 1 may be provided with only one tapered part 12, or may be provided with one tapered part 12 at both ends of the main body part 13, so that the support main body 1 can be inserted into the lumen from two directions. That is, the shape of the support main body 1 can be a large outer diameter in the middle and a small outer diameter at both ends, or a large outer diameter at one end and a small outer diameter at the other end. Specifically, the support main body 1 may be spherical, hemispherical, ellipsoidal, semi-ellipsoidal, conical, circular truncated cone, waterdrop shape, rugby, gourd, and the similar shape.

Specifically, in this embodiment, the support main body 1 is waterdrop shape, and the sharper end of the support main body 1 forms a tapered part 12, and the support main body 1 can be easily inserted into the lumen 101 by using the tapered part 12.

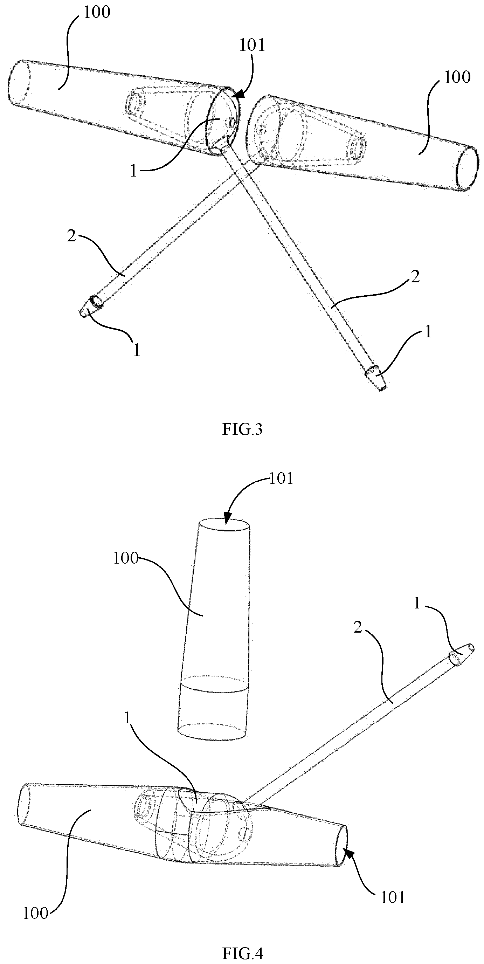

As shown in FIG. 3, an end-to-end anastomosis can be performed using two lumen anastomosis support dilators of this embodiment, using two lumen anastomosis support dilators, a support main body 1 is inserted into the lumen 101 through the end opening of a lumen 101 respectively, so as to support the lumen wall 100, with both support main bodies 1 having the same maximum outer diameter. The connecting rod 2 is located outside the lumen 101 which is used to adjust the position of the support main body 1 and to take out the support main body 1. The ends of the two lumens 101 are arranged opposite to each other for stitching, after about two-thirds of the stitching is completed, the support main body 1 is gripped by an instrument and taken out from the lumen 101, and the remaining parts are continued to be stitched until the operation is completed.

As shown in FIG. 4 , the end-to-side anastomosis can also be performed by using two lumen anastomosis support dilators of the present embodiment, two lumen anastomosis support dilators are used, and the support main body 1 of one lumen anastomosis support dilator is inserted into the end opening of one lumen 101 to support the lumen wall 100 (the lumen anastomosis support dilator is not shown in the figure), and the support main body 1 of the other lumen anastomosis support dilator is inserted into the side opening of the other lumen 101 to support the lumen wall 100, so that the end opening of one lumen 101 is stitched to the side opening of the other lumen 101. During the anastomosis procedure, the fluid in the lumen 101 can continue to flow through the hollow cavity 11. For the specific stitching process, reference may be made to the above-mentioned stitching process of end-to-end anastomosis, which will not be repeated here.

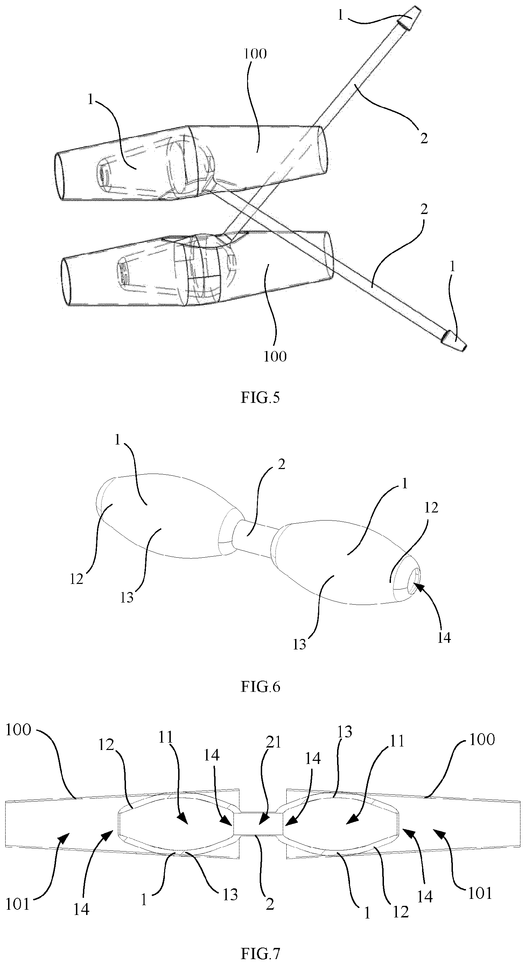

In addition, as shown in FIG. 5, two lumen anastomosis support dilators of this embodiment can also be used to perform side-to-side anastomosis, for details, see FIG. 5, two lumen anastomosis support dilators are used to insert a support main body 1 into lumen 101 through the lateral opening of lumen 101 to perform side-to-side anastomosis of two lumens 101. The specific stitch process can be referred to the above end-to-end anastomosis and end-to-side anastomosis, and will not be repeated here.

The connecting rod 2 may be provided with a plurality of support main bodies 1, so that an anastomosis between two lumens 101 can be performed by using one lumen anastomosis support dilator, and the main body parts 13 of the plurality of support main bodies 1 may have various different outer diameters, therefore, one lumen anastomosis support dilator can be applied to lumen 101 of different diameters. In this embodiment, a support main body 1 is provided at each end of the connecting rod 2, and the two support main bodies 1 can be placed in the two lumens 101 to be anastomosed respectively, so that the anastomosis of the two lumens 101 can be achieved by using one lumen anastomosis support dilator, which makes the two lumens 101 relatively fixed during stitching, which facilitates the stitching operation and reduces the number of equipment needed in the operation.

The two support main bodies 1 on the same connecting rod 2 may have the same maximum outer diameter, so as to be suitable for anastomosis between lumens 101 with the same diameter. In addition, the main body parts 13 of the two support main bodies 1 may also have different maximum outer diameters, so as to be suitable for anastomosis between lumens 101 with different diameters. In some embodiments, one or more support main bodies 1 may also be provided in the middle of the connecting rod 2 to meet the needs of different lumen anastomosis operations.

Since the openings of the two lumens to be stitched may have different relative positions, the included angle between the axis lines of the two support main bodies 1 at both ends of the connecting rod 2 can be 0-180°, so that the axis lines between the two support main bodies 1 can be set as collinear, parallel or other angles to adapt to the needs of lumen anastomosis operation under different conditions.

The connecting rod 2 can be bent, so that the relative positions between different support main bodies 1 on the connecting rod 2 can be adjusted to prevent damage to the lumen 101 when the support main body 1 is taken out, the connecting rod 2 can be bent at any angle to be suitable for different anastomosis operations. For specific anastomotic operations, the connection rod 2 may also be pre-molded into a curved tube, as in this embodiment, the angle of axial lines between the connection rod 2 and support main body 1 is 45°. Preferably, the connecting rod 2 can be made of an elastic material or a shape memory material, furthermore, the connecting rod 2 and the support main body 1 can be an integrally -formed structure, the connecting rod 2 is made of the same material as the support main body 1, which is in the shape of strip or column, and can be set in different lengths according to demand. The connecting rod 2 can be deformed compliantly to realize the shape of the lumen 101, while maintaining a certain angle so as to control the position and angle of the support main body 1.

The lumen anastomosis support dilator of this embodiment solves the tricky problems in lumen stitching through a simple and practical design, which can be easily supported and dilated during operation to provide the same caliber anastomosis end face more accurately, with simple operation and easy recycle. Only simple and special micro forceps, needle holders or similar instruments are needed to complete the placement and removal, which greatly improves the efficiency and success rate of stitching and reduces the risk of adverse outcomes. Moreover, the invention adopts integrally -forming process with relatively low cost, which is very conducive to the popular use of local and municipal hospitals with limited cost.

### Embodiment 2

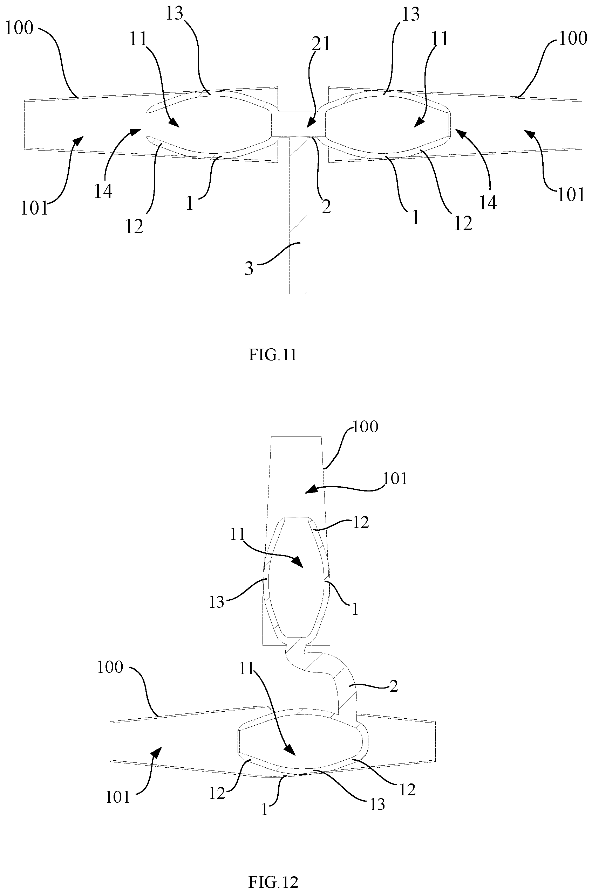

FIG. 6 to FIG. 11 are the lumen anastomosis support dilator of embodiment 2 of the invention. This embodiment is basically the same as embodiment 1, except that the two ends of the connecting rod 2 are respectively provided with a support main body 1 with an axial line on a straight line, and the axial lines of the two support main bodies 1 are collinear. Therefore, the lumen anastomosis support dilator in this embodiment may be suitable for end-to-end anastomosis (as shown in FIG. 7 and FIG. 11). During the operation, the support main body 1 is gripped with instruments (such as micro forceps, needle holders, etc.), and the sharper ends are placed in the forward direction, and placed into the end openings of the two lumen 101 to be stitched respectively, so as to obtain basically the same end face size and shape, and the connecting rod 2 is left outside the lumens 101. After about two-thirds of the stitching is completed, pull the connecting rod 2 outside the lumen 101 to cooperate with the instrument to grip the outer surface of the support main body 1, and take out the support main body 1 in the two lumen 101 respectively; then continue to stitch the remaining part until the operation is completed.

The connecting rod 2 may also be provided with a fluid channel 21, and both ends of the fluid channel 21 are communicated with the hollow cavity 11 of the support main body 1. The fluid in the lumen 101 can flow in the hollow cavity 11, and the fluid in the hollow cavity 11 can be drained into another lumen through the fluid channel 21.

Specifically, in this embodiment, one end of the hollow cavity 11 is connected to the fluid channel 21, since the end of the hollow cavity 11 is directly connected to the fluid channel 21, the fluid flowing into the hollow cavity 11 is completely discharged from the fluid channel 21, or the fluid in the cavity 11 is all injected from the fluid channel 21.

For example, in FIG. 7, both ends of the fluid channel 21 are respectively communicated with the ends of the hollow cavity 11 of a support main body 1, so the hollow cavities 11 of the two support main bodies 1 are communicated through the fluid channel 21. Different support main bodies 1 are arranged in different lumens 101, and the fluid in one lumen 101 can flow into the other lumen 101 through the hollow cavity 11 and the fluid channel 21, so that it can realize the different lumens 101 can be communicated with each other during the stitching operation, and the fluid flowing in the lumen 101 will not be interrupted during the operation.

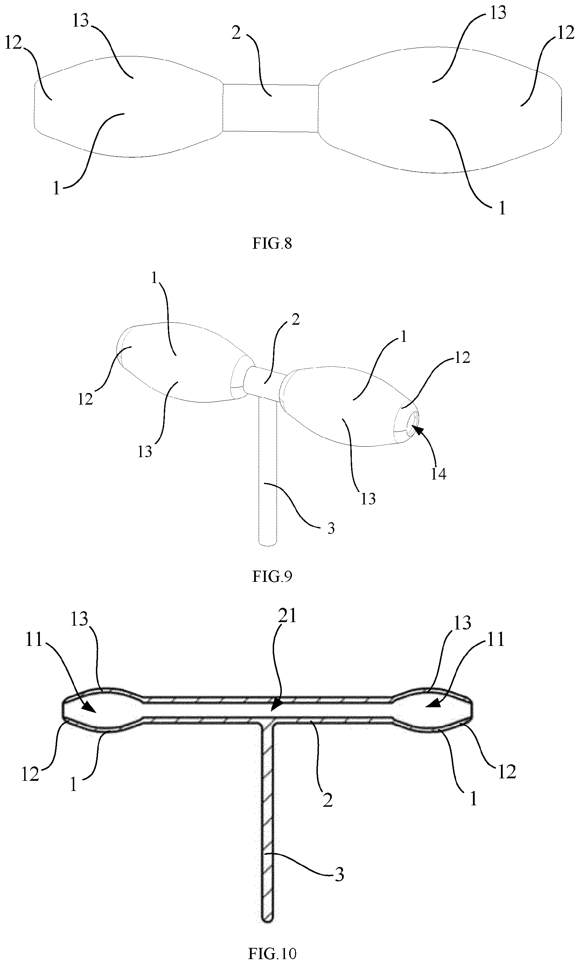

A plurality of support main bodies 1 on a lumen anastomosis support dilator can be the same size (as shown in FIG. 6, 7, and FIG. 9 to 11) or different sizes (as shown in FIG. 8). When the lumen anastomosis support dilator is provided with two support main bodies 1, one large and one small, the lumen anastomosis support dilator can be adapted to the lumen 101 with various diameters, so as to improve the application range of the lumen anastomosis support dilator and use different sizes of support main bodies 1 for different diameters of the lumen 101.

Therefore, an anastomosis operation can be performed without interrupting the flowing of internal substances. In an alternative embodiment, the hollow cavity 11 can also be closed at one end, closed at both ends, or a solid structure inside, and the support main body of the solid structure has a larger supporting force; or, the connecting rod 2 is not provided with a fluid channel 21 or is a solid structure, and the pushing and pulling force of the connecting rod of the solid structure is greater to prevent fluid in the lumen 101 from entering the lumen anastomosis support dilator, which is suitable for the situation where the flowing of substances in the lumen 101 needs to be interrupted during operation.

The lumen anastomosis support dilator further includes an operating rod 3, specifically, as shown in FIG. 9, the end of the operating rod 3 is connected to the middle of the connecting rod 2. The support main body 1 can be pushed and pulled by the operating rod 3 to adjust the position and angle of the support main body 1, and the user can use the operating rod 3 to take the support main body 1 out of the lumen 101.

### Embodiment 3

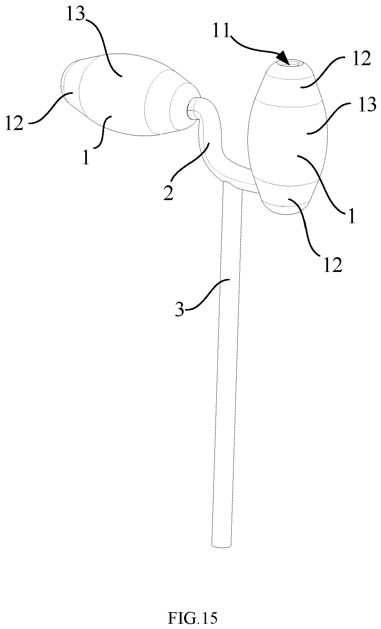

Figures 12 to 16 show the lumen anastomosis support dilator according to embodiment 3 of the present invention, this embodiment is basically the same as embodiment 1, the difference is that each end of the connecting rod 2 is provided with a support main body 1, and the axis lines of the two support main bodies 1 are perpendicular to each other. Therefore, when the lumen anastomosis support dilator of this embodiment is applied to end-to-side anastomosis, as shown in FIG. 12 for details, the connecting rod 2 is a curved tube, and the extension directions of the axis lines of the two support main bodies 1 on the connecting rod 2 are basically perpendicular to each other. Between the two lumens 101 to be anastomosed, wherein an opening is provided at the side of one lumen 101, one support main body 1 of the lumen anastomosis support dilator is inserted into the lumen 101 from the side opening, and the other support main body 1 is inserted into the end opening of the other lumen 101, the support main body 1 can be gripped by instruments (such as micro forceps, needle holders, etc.) to reduce its volume in the process of inserting, and at the same time the tapered shape 12 of the support main body 1 can be used as an advancing end to facilitate the entry of the support main body 1 into the lumen 101. After the stitching is partially completed, the support main body 1 can be removed from the unstitched gap, and then the entire stitching is completed.

The above-mentioned bending rod can be a bending rod obtained by direct processing and forming, and it can also be understood that the connecting rod 2 in the embodiment 2 is a bendable material, and the bending rod of this embodiment can be obtained by bending the straight rod in the embodiment 2.

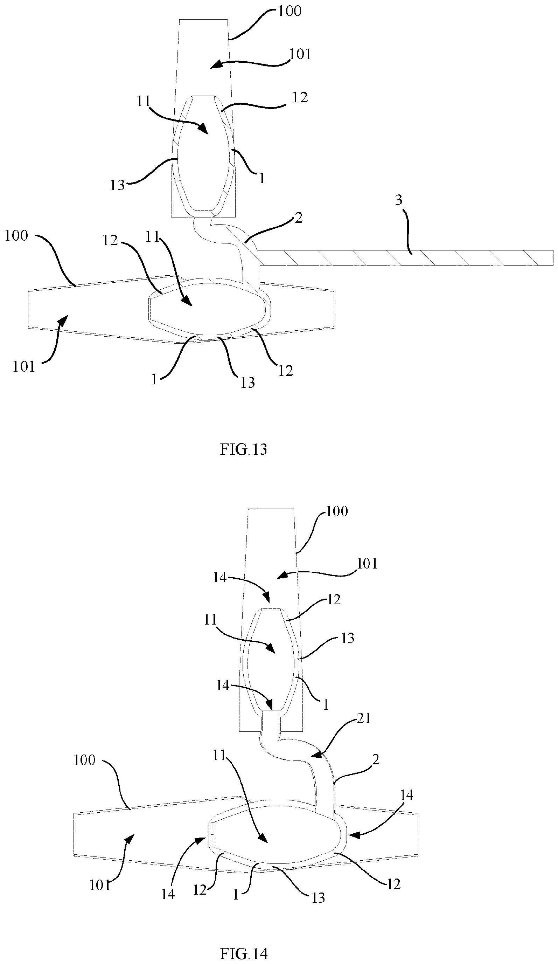

As shown in FIG. 14, in the lumen 101 with the side opening, since the support main body 1 has the hollow cavity 11, the fluid in the lumen 101 can flow normally. In addition, a fluid channel 21 may be provided in the connecting rod 2, and the fluid channel 21 can be communicated with the hollow cavities 11 of the two support main bodies 1, so that the fluid between the two lumens 101 can be communicated with each other. In the alternative embodiments, the fluid channel 21 may not be provided (as shown in FIG. 12 and FIG. 13), or the flowing direction of the fluid channel 21 and the hollow cavity 11 may be changed to meet different surgical needs.

The support main bodies 1 on the same lumen anastomosis support dilator can be the same outer diameter (as shown in FIG 12 and FIG 13), or can have different shapes and sizes (as shown in FIG 14 to FIG 16) to fit different lumens 101, those skilled in the art can make adjustments according to the actual situation.

As shown in FIG. 13, FIG. 15 and FIG. 16, the lumen anastomosis support dilator can also include an operating rod 3, and the end of the operating rod 3 is connected to the middle of the connecting rod 2, so as to facilitate the user to push and pull to adjust the position and angle of the support main body 1.

### Embodiment 4

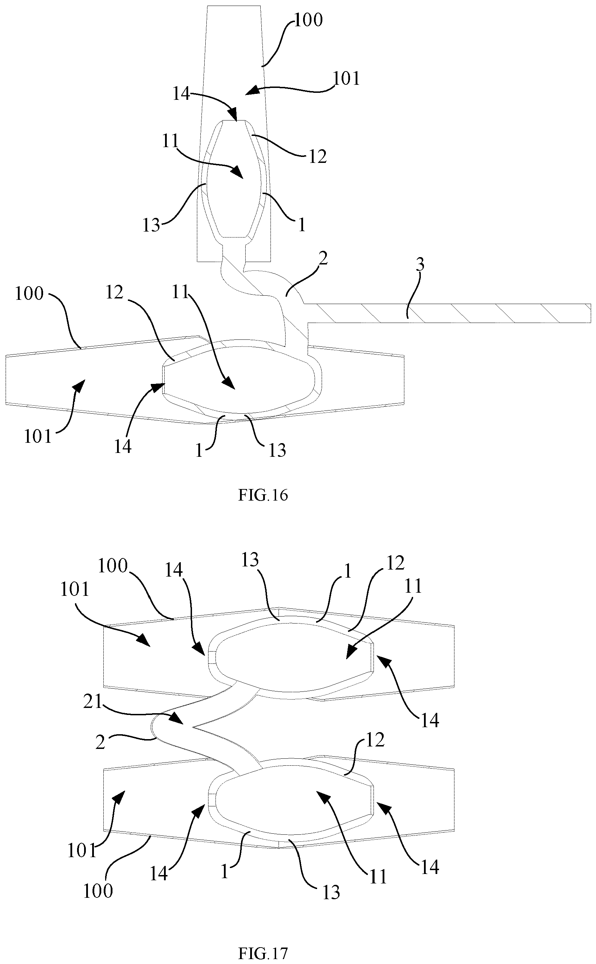

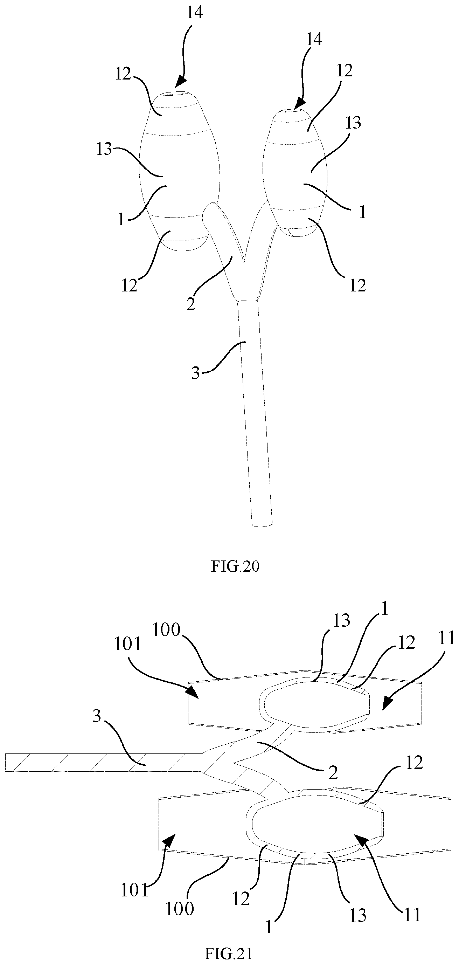

FIG. 17 to FIG. 21 show the lumen anastomosis support dilator of embodiment 4 of the invention, this embodiment is basically the same as embodiment 1, except that a support main body 1 is arranged at each end of the connecting rod 2, and the axis lines of the two support main bodies 1 are parallel to each other. Therefore, the lumen anastomosis support dilator of this embodiment can be applied to side-to-side anastomosis, the connecting rod 2 is V-shaped, and the two support main bodies 1 on the connecting rod 2 are arranged side by side. At this time, openings are both provided at the sides of the two lumen 101 to be anastomosed, and the two support main bodies 1 are respectively inserted into the two lumen 101 from the side openings, the inserting process can use instruments (such as micro forceps, needle holder, etc.) to grip the support main body 1 to reduce its volume, and at the same time use the tapered part 12 of the support main body 1 as the advancing end to facilitate the support main body 1 to enter the lumen 101. After the stitching is partially completed, the support main body 1 can be removed from the unstitched gap, and then the entire stitching is completed. The hollow cavity 11 of the support main body 1 prevents the fluid in the lumen 101 from being blocked, and the connecting rod 2 may also be provided with a fluid channel 21, both ends of the fluid channel are respectively communicated with the two hollow cavities 11 of the support main body 1, so that the fluid between the two lumens 101 can be communicated. In an alternative embodiment, the fluid channel 21 may not be provided, or the flow direction of the fluid channel 21 and the hollow cavity 11 may be changed to meet different surgical needs.

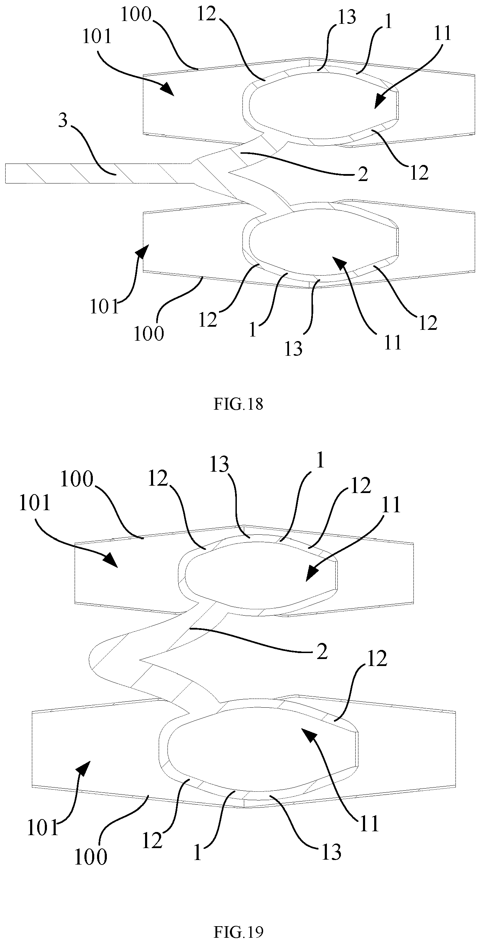

The support main bodies 1 on the same lumen anastomosis support dilator can have the same outer diameter (as shown in FIG. 17 and FIG. 18), and the main body part 13 of the support main body 1 can also have different shapes and sizes (as shown in FIG. 19 to FIG. 21), so as to adapt to different lumens 101, those skilled in the art can make adjustments according to actual conditions.

As shown in FIG. 18, FIG. 20 and FIG. 21, the lumen anastomosis support dilator can also include an operating rod 3, and the end of operating rod 3 is connected to the middle of the connecting rod 2, so as to facilitate the user to push and pull to adjust the position and angle of the support main body 1.

Although the specific embodiments of the invention are described above, a person skilled in the field should understand that this is only an example and that the scope of protection of the invention is limited by the attached claims. Those skilled in the art can make various changes or modifications to these embodiments without departing from the principle and essence of the present invention, but these changes and modifications all fall within the protection scope of the present invention.

## Claims

1. A lumen anastomosis support dilator, wherein the lumen anastomosis support dilator comprises:
a support main body, the support main body is made of an elastic material or a shape memory material and is used for partially placed in a lumen at least to dilate a lumen wall;
a connecting rod, the connecting rod is connected to the support main body.

2. The lumen anastomosis support dilator according to claim 1, wherein the connecting rod is provided with a plurality of support main bodies.

3. The lumen anastomosis support dilator according to claim 2, wherein the two ends of the connecting rod are each provided with the support main body.

4. The lumen anastomosis support dilator according to claim 2 or 3, wherein the support main body is a rotary body.

5. The lumen anastomosis support dilator according to claim 4, wherein the support main body comprises a main body part, at least one end of the main body part is provided with a tapered part, and the outer diameter of the tapered part decreases outward from the main body part, and the maximum outer diameter of the main body part is larger than the maximum outer diameter of the tapered part;
and/or, the included angle between the axis lines of the two support main bodies at both ends of the connecting rod is 0-180°.

6. The lumen anastomosis support dilator according to claim 4 or 5, wherein the maximum outer diameters of the main body parts of each support main body are same or different.

7. The lumen anastomosis support dilator according to at least one of claims 1 to 6, wherein the lumen anastomosis support dilator further comprises an operating rod, and the end of the operating rod is connected to the middle of the connecting rod.

8. The lumen anastomosis support dilator according to at least one of claims 1 to 7, wherein the connecting rod can be bent, so that the relative positions between the different support main bodies can be adjusted.

9. The lumen anastomosis support dilator according to at least one of claims 1 to 8, wherein the support main body is provided with a hollow cavity, and one end of the support main body is provided with a through hole communicating with the hollow cavity; or, two ends of the support main body are respectively provided with through holes communicating with the hollow cavity.

10. The lumen anastomosis support dilator according to claim 9, wherein the connecting rod is provided with a fluid channel, and the fluid channel is communicated with the hollow cavity of the support main body.
